# FASCICULE DE BREVET EUROPEEN

(11) **EP 0 964 871 B1**
(45) Date de publication et mention de la délivrance du brevet: **07.02.2007**
(21) Numéro de dépôt: 97950227.5
(22) Date de dépôt: 04.12.1997
(51) Int. Cl.: C07K 14/47, G01N 33/68

(54) **COMPOSES SYNTHETIQUES BIEPITOPIQUES UTILISABLES COMME ETALONS DANS LES DOSAGES BIOLOGIQUES DE LA TROPONINE I**
SYNTHETISCHE BIEPITOPISCHE ZUSAMMENSETZUNGEN ZUR VERWENDUNG ALS KALIBRATOREN BEI DER BIOLOGISCHEN BESTIMMUNG VON TROPONIN I
SYNTHETIC BI-EPITOPE COMPOUNDS USEFUL AS STANDARD MEASURE IN BIOLOGICAL DOSAGE OF TROPONIN I

(30) Priorité: 05.12.1996 FR 9614959
(43) Date de publication de la demande: 22.12.1999
(73) Titulaire: Bio-Rad Pasteur, 92430 Marnes-la-Coquette (FR)
(72) Inventeur: BABIN, Fabienne, Noëlle, F-78180 Montigny le Bretonneux (FR); CALZOLARI, Charles, Didier, F-69007 Lyon (FR); FLECHEUX, Odile, Suzanne, Hélène, F-78220 Viroflay (FR); GRANIER, Claude, F-34830 Clapiers (FR); LARUE, Catherine, Christiane, Marie, F-34000 Montpellier (FR); PAU, Bernard, Christian, F-34000 Montpellier (FR); RIEUNIER, François, Yves, F-78330 Fontenay le Fleury (FR); TRINQUIER, Sylvie, Marie-France, F-30320 Marguerittes (FR)
(74) Mandataire: Bernasconi, Jean Raymond
(86) Numéro de dépôt international: PCT/FR1997/002209
(87) Numéro de publication internationale: WO 1998/024816

(56) Documents cités:
- EP-A- 0 650 053
- EP-A- 0 752 426
- WO-A-94/27156
- WO-A-96/27661
- DE-A- 4 243 648
- MORJANA N A ET AL: "BIOCHEMICAL AND IMMUNOLOGICAL PROPERTIES OF A CYANOGEM BROMIDE FRAGMENT OF HUMAN CARDIAC TROPONIN I" FASEB JOURNAL, vol. 10, no. 6, 30 avril 1996, page A-1295 XP002028680
- LARUE C ET AL: "NEW MONOCLONAL ANTIBODIES AS PROBES FOR HUMAN CARDIAC TROPONIN I: EPITOPIC ANALYSIS WITH SYNTHETIC PEPTIDES" MOLECULAR IMMUNOLOGY, vol. 29, no. 2, 1 janvier 1992, pages 271-278, XP000400710
- VALLINS E.A.: "Molecular cloning of human cardiac troponin I using polymerase chain reaction" FEBS LETTERS, vol. 270, no. 1,2, septembre 1990, AMSTERDAM NL, pages 57-61, XP000470204 cité dans la demande

### LISTE DE SEQUENCES

(1) INFORMATION GENERALE:
   (i) DEPOSANT:
      (A) NOM: Pasteur Sanofi Diagnostics
      (B) RUE: 3 boulevard Raymond Poincaré
      (C) VILLE: Marnes la Coquette
      (E) PAYS: FRANCE
      (F) CODE POSTAL: 92430
      (G) TELEPHONE: 0153774000
      (H) TELECOPIE: 0153774133
   (ii) TITRE DE L' INVENTION: composés synthétiques biépitopiques utilisables comme étalons dans les dosages biologiques de la troponine I
   (iii) NOMBRE DE SEQUENCES: 29
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 4 acides aminés
      (B) TYPE: acide aminé
   (ii) TYPE DE MOLECULE: peptide
   (xi) DESCRIPTION DE LA SEQUENCE: SEQ ID NO: 1:
   Thr Glu Pro His
   1
(2) INFORMATION POUR LA SEQ ID NO: 2:
   (i) CARACTERISTIQUES DE LA SEQUENCE:
      (A) LONGUEUR: 4 acides aminés
      (B) TYPE: acide aminé
   (ii) TYPE DE MOLECULE: peptide
   (xi) DESCRIPTION DE LA SEQUENCE: SEQ ID NO: 2:
   His Ala Lys Lys
   1
(2) INFORMATION POUR LA SEQ ID NO: 3:
   (i) CARACTERISTIQUES DE LA SEQUENCE:
      (A) LONGUEUR: 7 acides aminés
      (B) TYPE: acide aminé
   (ii) TYPE DE MOLECULE: peptide
   (xi) DESCRIPTION DE LA SEQUENCE: SEQ ID NO: 3:
   Pro Ala Pro Ile Arg Arg Arg
   1               5
(2) INFORMATION POUR LA SEQ ID NO: 4:
   (i) CARACTERISTIQUES DE LA SEQUENCE:
      (A) LONGUEUR: 5 acides aminés
      (B) TYPE: acide aminé
   (ii) TYPE DE MOLECULE: peptide
   (xi) DESCRIPTION DE LA SEQUENCE: SEQ ID NO: 4:
   Leu Leu Gly Ala Arg
   1               5
(2) INFORMATION POUR LA SEQ ID NO: 5:
   (i) CARACTERISTIQUES DE LA SEQUENCE:
      (A) LONGUEUR: 4 acides aminés
      (B) TYPE: acide aminé
   (ii) TYPE DE MOLECULE: peptide
   (xi) DESCRIPTION DE LA SEQUENCE: SEQ ID NO: 5:
   Arg Lys Asn Ile
   1
(2) INFORMATION POUR LA SEQ ID NO: 6:
   (i) CARACTERISTIQUES DE LA SEQUENCE:
      (A) LONGUEUR: 4 acides aminés
      (B) TYPE: acide aminé
   (ii) TYPE DE MOLECULE: peptide
   (xi) DESCRIPTION DE LA SEQUENCE: SEQ ID NO: 6:
   Gln Lys Met Gln
   1
(2) INFORMATION POUR LA SEQ ID NO: 7:
   (i) CARACTERISTIQUES DE LA SEQUENCE:
      (A) LONGUEUR: 5 acides aminés
      (B) TYPE: acide aminé
   (ii) TYPE DE MOLECULE: peptide
   (xi) DESCRIPTION DE LA SEQUENCE: SEQ ID NO: 7:
   Gln Gly Pro Asp Asn
   1 5
(2) INFORMATION POUR LA SEQ ID NO: 8:
   (i) CARACTERISTIQUES DE LA SEQUENCE:
      (A) LONGUEUR: 4 acides aminés
      (B) TYPE: acide aminé
   (ii) TYPE DE MOLECULE: peptide
   (xi) DESCRIPTION DE LA SEQUENCE: SEQ ID NO: 8:
   Gly Pro Asp Asn
   1
(2) INFORMATION POUR LA SEQ ID NO: 9:
   (i) CARACTERISTIQUES DE LA SEQUENCE:
      (A) LONGUEUR: 10 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= repetition
         /note= "acides aminés 7 à 10 : (Gln Lys Met Gln)n avec n entier de 1 à 5"
   (xi) DESCRIPTION DE LA SEQUENCE: SEQ ID NO: 9:
   Ala Met Met Lys Ser Lys Gln Lys Met Gln
   1               5                   10
(2) INFORMATION POUR LA SEQ ID NO: 10:
   (i) CARACTERISTIQUES DE LA SEQUENCE:
      (A) LONGUEUR: 13 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= repetition
         /note= "acides aminés 10 à 13 : (Gln Lys Met Gln)n avec n entier de 1 à 5"
   (xi) DESCRIPTION DE LA SEQUENCE: SEQ ID NO: 10:
   Ala Lys Lys Ala Met Met Lys Ser Lys Gln Lys Met Gln
   1               5                   10
(2) INFORMATION POUR LA SEQ ID NO: 11:
   (i) CARACTERISTIQUES DE LA SEQUENCE:
      (A) LONGUEUR: 12 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= repetition
         /note= "acides aminés 7 à 10 : (Gln Lys Met Gln)n avec n entier de 1 à 5"
   (xi) DESCRIPTION DE LA SEQUENCE: SEQ ID NO: 11:
   Ala Met Met Lys Ser Lys Gln Lys Met Gln Gln Ala
   1               5                   10
(2) INFORMATION POUR LA SEQ ID NO: 12:
   (i) CARACTERISTIQUES DE LA SEQUENCE:
      (A) LONGUEUR: 15 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= repetition
         /note= "acides aminés 10 à 13 : (Gln Lys Met Gln)n avec n entier de 1 à 5"
   (xi) DESCRIPTION DE LA SEQUENCE: SEQ ID NO: 12:
   Ala Lys Lys Ala Met Met Lys Ser Lys Gln Lys Met Gln Gln Ala
   1               5                   10                  15
(2) INFORMATION POUR LA SEQ ID NO: 13:
   (i) CARACTERISTIQUES DE LA SEQUENCE:
      (A) LONGUEUR: 10 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= repetition
         /note= "acides aminés 4 à 7 : (Gln Lys Met Gln)n avec n entier de 1 à 5"
   (xi) DESCRIPTION DE LA SEQUENCE: SEQ ID NO: 13:
   Lys Ser Lys Gln Lys Met Gln Ala Met Met
   1               5                   10
(2) INFORMATION POUR LA SEQ ID NO: 14:
   (i) CARACTERISTIQUES DE LA SEQUENCE:
      (A) LONGUEUR: 13 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= repetition
         /note= "acides aminés 7 à 10 : (Gln Lys Met Gln)n avec n entier de 1 à 5"
   (xi) DESCRIPTION DE LA SEQUENCE: SEQ ID NO: 14:
   Ala Lys Lys Lys Ser Lys Gln Lys Met Gln Ala Met Met
   1               5                   10
(2) INFORMATION POUR LA SEQ ID NO: 15:
   (i) CARACTERISTIQUES DE LA SEQUENCE:
      (A) LONGUEUR: 12 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= repetition
         /note= "acides aminés 4 à 7 : (Gln Lys Met Gln)n avec n entier de 1 à 5"
   (xi) DESCRIPTION DE LA SEQUENCE: SEQ ID NO: 15:
   Lys Ser Lys Gln Lys Met Gln Ala Met Met Gln Ala
   1               5                   10
(2) INFORMATION POUR LA SEQ ID NO: 16:
   (i) CARACTERISTIQUES DE LA SEQUENCE:
      (A) LONGUEUR: 15 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= repetition
         /note= "acides aminés 7 à 10 : (Gln Lys Met Gln)n avec n entier de 1 à 5"
   (xi) DESCRIPTION DE LA SEQUENCE: SEQ ID NO: 16:
   Ala Lys Lys Lys Ser Lys Gln Lys Met Gln Ala Met Met Gln Ala
   1               5                   10                  15
(2) INFORMATION POUR LA SEQ ID NO: 17 :
   (i) CARACTERISTIQUES DE LA SEQUENCE:
      (A) LONGUEUR: 6 acides aminés
      (B) TYPE: acide aminé
   (ii) TYPE DE MOLECULE: peptide
   (xi) DESCRIPTION DE LA SEQUENCE: SEQ ID NO: 17 :
   Ala Lys Lys Ala Met Met
   1               5
(2) INFORMATION POUR LA SEQ ID NO: 18 :
   (i) CARACTERISTIQUES DE LA SEQUENCE:
      (A) LONGUEUR: 5 acides aminés
      (B) TYPE: acide aminé
   (ii) TYPE DE MOLECULE: peptide
   (xi) DESCRIPTION DE LA SEQUENCE: SEQ ID NO 18 :
   Ala Met Met Gln Ala
   1               5
(2) INFORMATION POUR LA SEQ ID NO: 19 :
   (i) CARACTERISTIQUES DE LA SEQUENCE:
      (A) LONGUEUR: 8 acides aminés
      (B) TYPE: acide aminé
   (ii) TYPE DE MOLECULE: peptide
   (xi) DESCRIPTION DE LA SEQUENCE: SEQ ID NO: 19 :
   Ala Lys Lys Ala Met Met Gln Ala
   1               5
(2) INFORMATION POUR LA SEQ ID NO: 20 :
   (i) CARACTERISTIQUES DE LA SEQUENCE:
      (A) LONGUEUR: 4 acides aminés
      (B) TYPE: acide aminé
   (ii) TYPE DE MOLECULE: peptide
   (xi) DESCRIPTION DE LA SEQUENCE: SEQ ID NO: 20:
   Pro Gly Asn Ser
   1
(2) INFORMATION POUR LA SEQ ID NO: 21 :
   (i) CARACTERISTIQUES DE LA SEQUENCE:
      (A) LONGUEUR: 6 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= insertion
         /note= "entre l'acide aminé 3 et l'acide aminé 4, groupement -NH-(CH₂)ₘ-CO avec m entier de 1 à 10"
   (xi) DESCRIPTION DE LA SEQUENCE: SEQ ID NO: 21 :
   Ala Lys Lys Ala Met Met
   1 5
(2) INFORMATION POUR LA SEQ ID NO: 22 :
   (i) CARACTERISTIQUES DE LA SEQUENCE:
      (A) LONGUEUR: 5 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= insertion
         /note= "côté N-terminal de l'acide aminé 1, groupement - NH-(CH₂)ₘ-CO avec m entier de 1 à 10"
   (xi) DESCRIPTION DE LA SEQUENCE: SEQ ID NO: 22 :
   Ala Met Met Gln Ala
   1 5
(2) INFORMATION POUR LA SEQ ID NO: 23 :
   (i) CARACTERISTIQUES DE LA SEQUENCE:
      (A) LONGUEUR: 8 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= insertion
         /note= "entre l'acide aminé 3 et l'acide aminé 4, groupement -NH-(CH₂)ₘ-CO avec m entier de 1 à 10"
   (xi) DESCRIPTION DE LA SEQUENCE: SEQ ID NO: 23 :
   Ala Lys Lys Ala Met Met Gln Ala
   1 5
(2) INFORMATION POUR LA SEQ ID NO: 24 :
   (i) CARACTERISTIQUES DE LA SEQUENCE:
      (A) LONGUEUR: 27 acides aminés
      (B) TYPE: acide aminé
   (ii) TYPE DE MOLECULE: peptide
   (ix) CARACTERISTIQUE ADDITIONELLE:
      (D) AUTRES RENSEIGNEMENTS: /label= insertion
         /note= "éventuellement entre l'acide aminé 14 et l'acide aminé 15, groupement -NH-(CH₂)ₘ-CO avec m entier de 1 à 10"
   (xi) DESCRIPTION DE LA SEQUENCE: SEQ ID NO: 24 :
   Gly Asn Tyr Arg Ala Tyr Ala Thr Glu Pro His Ala Lys Lys Ala Met
   1               5                   10                  15
   Met Gln Ala Leu Leu Gly Ala Arg Ala Lys Glu
               20                  25
(2) INFORMATION POUR LA SEQ ID NO: 25 :
   (i) CARACTERISTIQUES DE LA SEQUENCE:
      (A) LONGUEUR: 33 acides aminés
      (B) TYPE: acide aminé
   (ii) TYPE DE MOLECULE: peptide
   (xi) DESCRIPTION DE LA SEQUENCE: SEQ ID NO: 25 :
   Asn Tyr Arg Ala Tyr Ala Thr Glu Pro His Ala Lys Lys Lys Ser Lys
   1               5                   10                  15
   Gln Lys Met Gln Ala Met Met Gln Ala Leu Leu Gly Ala Arg Ala Lys
               20                  25                  30
   Glu
(2) INFORMATION POUR LA SEQ ID NO: 26 :
   (i) CARACTERISTIQUES DE LA SEQUENCE:
      (A) LONGUEUR: 35 acides aminés
      (B) TYPE: acide aminé
   (ii) TYPE DE MOLECULE: peptide
   (xi) DESCRIPTION DE LA SEQUENCE: SEQ ID NO: 26 :
   Gly Gly Asn Tyr Arg Ala Tyr Ala Thr Glu Pro His Ala Lys Lys Lys
   1               5                   10                  15
   Ser Lys Gln Lys Met Gln Ala Met Met Gln Ala Leu Leu Gly Ala Arg
               20                  25                  30
   Ala Lys Glu
           35
(2) INFORMATION POUR LA SEQ ID NO: 27 :
   (i) CARACTERISTIQUES DE LA SEQUENCE:
      (A) LONGUEUR: 39 acides aminés
      (B) TYPE: acide aminé
   (ii) TYPE DE MOLECULE: peptide
   (xi) DESCRIPTION DE LA SEQUENCE: SEQ ID NO: 27 :
   Gly Gly Asn Tyr Arg Ala Tyr Ala Thr Glu Pro His Ala Lys Lys Lys
   1               5                   10                  15
   Ser Lys Gln Lys Met Gln Gln Lys Met Gln Ala Met Met Gln Ala Leu
               20                  25                  30
   Leu Gly Ala Arg Ala Lys Glu
           35
(2) INFORMATION POUR LA SEQ ID NO: 28 :
   (i) CARACTERISTIQUES DE LA SEQUENCE:
      (A) LONGUEUR: 38 acides aminés
      (B) TYPE: acide aminé
   (ii) TYPE DE MOLECULE: peptide
   (xi) DESCRIPTION DE LA SEQUENCE: SEQ ID NO: 28 :
   Gly Asn Tyr Arg Ala Tyr Ala Thr Glu Pro His Ala Lys Lys Lys Ser
   1               5                   10                  15
   Lys Gln Lys Met Gln Gln Lys Met Gln Ala Met Met Gln Ala Leu Leu
               20                  25                  30
   Gly Ala Arg Ala Lys Glu
           35
(2) INFORMATION POUR LA SEQ ID NO: 29 :
   (i) CARACTERISTIQUES DE LA SEQUENCE:
      (A) LONGUEUR: 24 acides aminés
      (B) TYPE: acide aminé
   (ii) TYPE DE MOLECULE: peptide
   (xi) DESCRIPTION DE LA SEQUENCE: SEQ ID NO: 29 :
   Arg Pro Ala Pro Ala Pro Ile Arg Arg Arg Pro Gly Asn Ser Thr Glu
   1               5                   10                  15
   Pro His Ala Lys Lys Lys Ser Lys
               20

## Description

La présente invention concerne des composés synthétiques biepitopiques utilisables comme étalons dans les immunoessais pour le dosage de la troponine I leur procédé de préparation ces compositions et des trousses contenant de tels composés ainsi que des procédés d'immunodosages mettant en oeuvre de tels composés.

On sait que la troponine est un complexe protèique myofibrillaire constitué de trois protéines, les troponines I, T et C Ce complexe protéique permet ce contribuer à la régulation de la contraction du muscle par l'ion Ca²⁻ en interagissant avec la myosine et l'actine. De façon plus précise, on sait que lorsqu un influx nerveux arrive au niveau de la plaque motrice d'un muscle, il y a génération d'un potentiel d'action qui est transmis au réticulum sarcoplasmique. Le Ca²⁻ est alors libéré dans le cytosol et se fixe sur la troponine C ce qui entraine un renforcement de l'interaction entre la troponine I et la troponine C et par suite un changement de conformation du complexe troponine I, T, C. Il y a alors libération des sites d'interaction actine-myosine, ce qui permet le mouvement de contraction du muscle.

Lorsque le muscle est endommagé, que ce soit le muscle cardiaque lors d'une nécrose myocardique consécutive à un infarctus du myocarde ou que ce soit le muscle squelettique lors d'efforts physiques prolongés, les troponines alors libérées apparaissent plus ou moins rapidement dans la circulation sanguine.

Ainsi on a récemment préconisé le dosage de la troponine pour le diagnostic précoce de l'infarctus du myocarde que ce soit celui de la troponine T dans *Circulation (1991,)* 83, *pp. 902-912,* ou de la troponine 1 *dans Am Heart J* (1987), *110, pp. 1333-1344,* et *Molecular Immunology* (1992), *29(2)*, *pp*. *271-278.* De même, on a proposé le dosage de la troponine T cardiaque pour mesurer le succès de la thérapie thrombolytique suite à un infarctus du myocarde dans *Br. Heart J., (1994)*, *71 pp. 242-248,* ainsi que le dosage de la troponine squelettique pour la mesure du dommage des muscles squelettiques (abrégé n° 35 de *l'American Association for Clinical Chemistry, 46th National Meeting, New Orleans, July 17-21, 1994).* Il est a noter que le dosage des différentes troponines cardiaques et squelettiques est aujourd'hui un moyen très utile pour le diagnostic de pathologies humaines et animales.

Il est bien connu que les immunoessais pratiqués dans les laboratoires d'analyses biologiques nécessitent la fourniture par le fabricant, à côté des réactifs nécessaires au dosage (c'est-à-dire des anticorps marqués ou non des agents de révélation et des solutions ce dilution) d'un étalon du composé à déterminer qui mis en oeuvre dans des conditions analogues à celles de l'échantillon à étudier servira de référence pour le calcul des résultats et/ou de témoin positif.

Pour obtenir l'étalon et/ou le témoin du composé à déterminer on peut utiliser ledit composé purifié sous forme lyophilisée (accompagné dd'un solvant dans lequel le composé sera dissous par l'utilisateur avant l'emploi) ou prêt à l'emploi.

Du fait que les réactifs biologiques sont instables les solutions étalons ou témoins préparées à partir de lyophilisât sont congelées en doses unitaires et conservées à -80°C. On a constaté par ailleurs que ces solutions n'étaient pas stables plus de quelques heures à +4°C, même si des inhibiteurs de protéases ou des agents antibactériens y étaient ajoutés. Ceci oblige donc les utilisateurs à préparer extemporanément leurs solutions d'étalonnage.

La demande de brevet publiée sous le numéro FR-A- 2 701 954 divulgue une composition stabilisée de troponine I ou T pour immunoessai caractérisée en ce qu'elle est constituée d'une solution aqueuse contenant de la troponine I ou de la troponine T, en mélange avec de la troponine C et notamment dans des proportions de 1 à 10 équivalents molaires de troponine C par équivalent de troponine I ou T, et du chlorure de calcium. Cette technique permet la conservation durant plusieurs jours à +4°C des solutions étalons, plus ou moins diluées, de troponine I ou T.

La demande de brevet publiée sous le numéro FR 2 734 267 décrit ces solutions étalons de la troponine composées d'un complexe ternaire formé par la troponine I, la troponine T et la troponine C.

Les matières premières utilisées pour obtenir ces étalons sont d origine humaine ou animale et les étalons ou témoins ainsi obtenus sont stables pendant environ un mois à +4°C.

La demande WO 94/15217 décrit certains peptides synthétiques utiles comme immunogènes pour la préparation des anticorps reconnaissant le peptide N-terminal de la troponine I. Certains de ces peptides peuvent être utilises comme étalons dans des immunodosages de la troponine I, mettant en oeuvre les anticorps objets de l'invention couverte par la demande WO 94/15217

De même, la demande de brevet WO 94/27156 porte sur un procédé de dosage de la troponine I cardiaque, mettant en oeuvre des anticorps spécifiques de la troponine I cardiaque. Ces anticorps peuvent être préparés à partir de fragments peptidiques ayant une séquence absente de la troponine I du muscle squelettique et donc spécifique de la troponine I cardiaque. Toutefois, cette demande ne divulgue ni ne suggère la possibilité d'utiliser certains fragments peptidiques comme étalons dans les immunodosages de la troponine I

On sait également que la demande WO 96127661 décrit des solutions aqueuses pour stabiliser des protéines et des peptides. Ces solutions trouvent notamment leur application dans les tests diagnostiques de protéines ou peptides

Selon WO 97/27661, ces solutions aqueuses permettent même d'augmenter la stabilité des fragments de la troponine I qui sont connus pour être moins stables que la troponine I entière.

La demande EP-A-752 426 publiée le 8 janvier 1997 décrit aussi des étalons de la troponine I composés d'un ou de plusieurs peptides fixés sur une molécule porteuse telles que des protéines de haut poids moléculaire (>100 KD) ou des polymères.

La demande EP-A-650 053 décrit des étalons synthétiques contenant des sites actifs pour un ou plusieurs récepteurs, reliés entre eux avec une structure arborescente. Cette demande décrit plus spécialement des étalons synthétiques de la troponine T qui ne sont stables en solution que pendant 3 semaines.

Il a été maintenant trouvé qu'il est possible d'obtenir des étalons synthétiques utilisables pour l'évaluation de la troponine I qui sont stables pendant plusieurs mois.

En effet, la structure particulière des composés de l'invention leur confère une excellente stabilité.

Les composés de la présente invention permettent donc d'obtenir des étalons hautement standardisés, stables en solution pendant plusieurs mois et d'éviter les étapes de purifications et les procédés complexes d'extraction nécessaires pour la préparation des étalons de la troponine I à partir des organes animaux.

Il est décrit des composés qui comprennent deux épitopes de la troponine I, reliés entre eux par un groupe de liaison (« linker ») et répondent à la formule générale I

Ξ - E₁ - Z -E₂ - Ψ (I)

dans laquelle
- E₁, E₂, identiques ou différents représentent une séquence peptidique comprenant soit un épitope minimum de la troponine I, soit un épitope étendu,
- Z représente
   - une séquence peptidique de 1 à 40 acides aminés (aa), à condition toutefois que - E₁ - Z - E₂ - ne forment pas ensemble une partie de la séquence de la troponine I.
   - une chaine aminoalkyl(C₁-C₁₀)carbonyle linéaire ou ramifiée, ou
   - une construction mixte constituée d'au moins une séquence peptidique ce 1 à 10 acides aminés et au moins une chaine aminoalkyl(C₁-C₁₀)carbonyle linéaire ou ramifiée,
- Ξ représente
   - un atome d hydrogène un groupement acétyle, une séquence peptidique de 1 à 10 acides aminés, une séquence peptidique de 1 à 10 acides aminés N-α acétylée, un groupement cystéinyle, biotinyle ou biocytinyle, une séquence peptidique de 1 à 10 acides aminés ponant un reste cystémyle biotinyle ou biocytinyle,
   - une chaîne aminoalkyl(C₁-C₁₀)carbonyle linéaire ou ramifiée
   - une chaîne aminoalkyl(C₁-C₁₀)carbonyle linéaire ou ramifiée N-α-acètylée
   - une construction mixte constituée d'au moins une séquence peptidique ce 1 à 10 acides aminés et au moins une chaîne aminoalkyl(C₁-C₁₀)carbonyle linéaire ou ramifiée, ou
   - une construction mixte constituée d'au moins une séquence peptidique ce 1 à 10 acides aminés et au moins une chaine aminoalkyl(C₁-C₁₀)carbonyle linéaire ou ramifiée, portant un reste biotinyle, biocytinyle ou cystéinyle.
- Ψ représente
   - un radical hydroxyle, un radical amino, une séquence peptidique de 1 à 10 acides aminés, une séquence peptidique de 1 à 10 acides aminés portant un groupement amino terminal,
   - une chaîne aminoalkyl(C₁-C₁₀)carbonyle linéaire ou ramifiée,
   - une chaîne aminoalkyl(C₁-C₁₀)carbonyle linéaire ou ramifiée portant un radical hydroxyle ou un radical amino,
   - une construction mixte constituée d'au moins une séquence peptidique ce 1 à 10 acides aminés et au moins une chaîne aminoalkyl(C₁-C₁₀)carbonyle linéaire ou ramifiée.
   - une construction mixte constituée d'au moins une séquence peptidique ce 1 à 10 acides aminés et au moins une chaine aminoalkyl(C₁-C₁₀)carbonyle linéaire ou ramifiée, portant un radical hydroxyle ou un radical amino.

On considère que - E₁ - Z - E₂ - n'est pas une partie de la séquence de la troponine I, lorsque - E₁ - Z - E₂ - diffère d'un fragment donné de la séquence de la troponine I par substitution, délétion ou insertion non conservative d'au moins un acide aminé, de préférence de 2 acides aminés, plus particulièrement de 5 acides aminés.

Z peut notamment représenter
- une chaine de formule II :

   -NH-(CH₂)ₘ-CO- (II)

   dans laquelle m représente un nombre entier de 1 à 10.
- ou une construction mixte de formules III à V

   -[pep₁-NH-(CH₂)ₘ-CO]p- (III)

   -[pep₁-NH-(CH₂)ₘ-CO-pep₂]ₚ- (IV)

   -[NH-(CH₂)m-CO-pep₂]ₚ- (V)

   dans lesquelles
   - m représente un nombre entier de 1 à 10,
   - p représente un nombre entier de 1 à 5, et
   - pep₁ et pep₂ identiques ou différents représentent une chaine peptidique comportant de 2 à 10 acides aminés
   Ξ peut notamment représenter
- une chaîne de formule IIa

   -NH-(CH₂)_{q}-CO- (IIa)

   dans laquelle q représente un nombre entier de 1 à 10.
- ou une construction mixte de formules IIIa à Va

   -[pep₃-NH-(CH₂)_{q}-CO]ᵣ- (IIIa)

   -[pep₃-NH-(CH₂)_{q}- CO-pep₄]ᵣ- (IVa)

   -[NH-(CH₂)_{q}-CO-pep₄]ᵣ- (Va)

   dans lesquelles
   - q représente un nombre entier de 1 à 10,
   - r représente un nombre entier de 1 à 5,
      et
   - pep₃ et pep₄ identiques ou différents représentent une chaine peptidique comportant de 2 à 10 acides aminés,
- une chaîne de formules IIa à Va N-α acétylée, ou une cnaine de formules IIa à Va portant un reste biotinyle, biocytinyle ou cystéinyle ;
   et Ψ peut notamment représenter
- une chaine de formule IIb :

   -NH-(CH₂)ₛ-CO- (IIb)

   dans laquelle s représente un nombre entier de 1 à 10,
- ou une construction mixte de formules IIIb à Vb

   -[pep₅-NH-(CH₂)ₛ-CO]ₜ- (IIIb)

   -[pep₅-NH-(CH₂)₅- CO-pep₆]ₜ- (IVb)

   -[NH-(CH₂)₅-CO-pep₆]ₜ- (Vb)

   dans lesquelles
   - s représente un nombre entier de 1 à 10,
   - t représente un nombre entier de 1 à 5,
      et
   - pep₅ et pep₆ identiques ou différents représentent une chaine peptidique comportant de 2 à 10 acides aminés,
- une chaîne de formules IIb à Vb portant un radical hydroxyle ou un radical amino sont également décrits des composés dans
lesquels Ξ représente soit une chaine de formule IIa, soit une construction mixte IIIa à Va, soit une chaine de formules IIa à Va N-α-acétylée ou une chaîne ce formules IIa à Va portant un reste biotinyle, biocytinyle ou cystéinyle
sont également décrits des composés dans lesquels Ψ représente soit une chaîne de formule IIb soit une construction mixte de formules IIIb à Vb, soit une chaîne de formules IIb à Vb portant un radical hydroxyle ou un radical amino.
Z peut représenter soit une chaîne de formule II, soit une construction mixte de formules III à V

Les séquences peptidiques comprenant un épitope minimum de la troponine I ont été déterminées à l'aide des anticorps monoclonaux anti-troponine I. De tels anticorps sont décrits par Larue et al. *(Molec. Immunology, (1992)*, *vol 20 n° 29, pp. 271-278),* Bodor et al. *(Clin. Chem. (1992), vol 38 n° 11 pp. 2203-2214),* Granier et al *(Protein Science, (1997)*, *vol 6 suppl* 1 *p*. *61)* et dans la demande WO 94/15217. La majorité de ces anticorps sont disponibles dans le commerce (Hytest LTD-Turku, Finland).

Pour déterminer la séquence peptidique comprenant un épitope minimum de la troponine I, il a été procédé à la synthèse simultanée de peptides ce 10 acides aminés, dont la séquence recouvre la séquence du peptide précédent par acides aminés, selon le schéma suivant :
- Peptide 1 = acides aminés 1 - 10
- Peptide 2 = acides aminés 4 - 13
- Peptide 3 = acides aminés 7 - 16
- etc.

Dans leur ensemble, les peptides synthétisés et testés décrivent la totalité de la séquence de la protéine *(Vallins J. et al., FEBS Letters (1990)*, *vol 270 n° 1-2*, *pp. 57-61).*

Il a ensuite été procédé à l'identification des peptides réagissant avec chacun des anticorps monoclonaux utilisés, par un test immunoenzymatique ce liaison de chaque peptide avec chaque anticorps.

Il est indiqué par exemple ci-dessous, pour certains anticorps monoclonaux anti-troponine I, la séquence peptidique comprenant un épitope minimum immunologiquement réactif pour chaque anticorps et la position ce cette séquence peptidique dans la séquence de la troponine I, telle que décrite dans *FEBS Letters* (*1990*), *vol. 270 n° 1-2*, *pp.* 57-61.

| **Anticorps** | **Epitope*** | **Position dans la séquence de la troponine I** |
|---|---|---|
| 5C3 | DAARE | 7-11 |
| 10B11 | PAPIRRR | 16-22 |
| HC11 | RRRSS | 20-24 |
| HG5 et HC2 | YRAYATEP | 26-33 |
| 11 E 12 | TEPH | 31-34 |
| 10F4 | HAKK | 34-37 |
| HC7 | ISASRKLOLK | 41-50 |
| H414 | AKQELE | 57-62 |
| 5F1 | AELQ | 91-94 |
| 13H7 et 20C5 | LODLCR | 93-98 |
| 2B7 | DLTQKIFDLR | 127-136 |
| 3G11 et 15F2 | PTLRRVR | 142-148 |
| 2E6 et 8G2 | ADAMMQA | 151-157 |
| 7D1 | ALLG | 157-160 |
| 8E1 | LLGAR | 158-162 |
| 9E8 | SLDLRAH | 166-172 |
| 4B5 | ENRE | 184-187 |
| 7F4 | OWRKNID | 190-196 |

| | | |
|---|---|---|
| * Les séquences peptidiques sont indiquées en utilisant un code à une lettre. | | |

Les séquences peptidiques dérivant desdites séquences peptidiques par substitution, délétion ou insertion d'un acide aminé, dans la mesure où elles présentent une affinité de liaison à l'anticorps équivalente, sont aussi décrites.

Les séquences linéaires E₁ et/ou E₂ n° 1 à 5 suivantes sont décrites.
séquence n° 1 : -Thr Glu Pro His-
   soit
   -TEPH-
séquence n° 2 : -His Ala Lys Lys-
   soit
   -HAKK-
séquence n° 3 : -Pro Ala Pro Ile Arg Arg Arg-
   soit
   -PAPIRRR-
séquence n° 4 : -Leu Leu Gly Ala Arg-
   soit
   -LLGAR-
séquence n° 5 : -Arg Lys Asn Ile-
   soit
   -RKNI-.

Les séquences ci-dessus sont données à titre d'exemple non limitatif.

Lorsque le linker Z comporte une séquence peptidique, celle-ci peut former avec les séquences E₁ et/ou E₂ un épitope étendu de la troponine I. Cet épitope étendu peut notamment avoir pour séquence -TEPHAKK- ou -QALLGAR- ou -PTLRRVRISA- ou -GKFKRPTLRRVR- ou -LGFAELQD- ou -NYRAYATEPH- ou -AYATEPH- ou -LGFAELQ-, etc.

Ces épitopes étendus, qui amplifient la réponse immunologique pour les anticorps ont été déterminés en ajoutant des acides aminés à la séquence peptidique comprenant un épitope minimum déterminé comme décrit précédemment.

Comme il a été mentionné ci-dessus, la séquence peptidique du linker Z peut comporter de 1 à 40 acides aminés. On préfère que le nombre d'acides aminés qui forment la séquence peptidique de Z soit inférieur à 30 plus particulièrement inférieur à 20.

Une séquence peptidique de Z peut inclure une séquence choisie parmi les séquences d'acides aminés suivantes :
- Gln Lys Met Gln- soit -QKMQ-.
- Gly Pro Asp Asn- soit -GPDN-,
- Ala Met Met- soit -AMM-,
- Ala Lys Lys- soit -AKK-.
- Lys Ser Lys- soit -KSK- et
- Pro Gly Asn Ser- soit -PGNS-.

Cette séquence peptidique de Z particulièrement préférée peut contenir un enchaînement d'acides aminés répété et a un nombre d'acides aminés inférieur à 30.

A titre d'exemple, sont données les séquences peptidiques Z de formules suivantes dans lesquelles n représente un nombre entier de 1 à 5
séquence n° 6 : -Gln Lys Met Gln-
   soit
   -QKMQ-
séquence n° 7 : -Gln Gly Pro Asp Asn-
   soit
   -QGPDN-
séquence n° 8 : -Gly Pro Asp Asn-
   soit
   -GPDN-
séquence n° 9 : -Ala Met Met Lys Ser Lys (Gln Lys Met Gln)ₙ-
   soit
   -AMMKSK-(QKMQ)ₙ-
séquence n° 10 : -Ala Lys Lys Ala Met Met Lys Ser Lys-(Gln Lys Met Gln)ₙ-
   soit
   -AKKAMMKSK-(QKMQ)ₙ-
séquence n° 11 : -Ala Met Met Lys Ser Lys-(Gln Lys Met Gln)ₙ-Gln Ala-
   soit
   -AMMKSK-(QKMQ)ₙ-QA-
séquence n° 12: -Ala Lys Lys Ala Met Met Lys Ser Lys-(Gln Lys Met Gln)ₙ-Gln Ala-
   soit
   -AKKAMMKSK-(QKMQ)ₙ-QA-
séquence n° 13 : -Lys Ser Lys-(Gln Lys Met Gln)ₙ-Ala Met Met-
   soit
   -KSK-(QKMQ)ₙ-AMM-
séquence n° 14. -Ala Lys Lys Lys Ser Lys-(Gln Lys Met Gln)ₙ-Ala Met Met-
   soit
   -AKKKSK-(QKMQ)ₙ-AMM-
séquence n° 15 : -Lys Ser Lys-(Gln Lys Met Gln)ₙ-Ala Met Met Gln Ala-
   soit
   -KSK-(QKMQ)ₙ-AMMQA-
séquence n° 16: -Ala Lys Lys Lys Ser Lys-(Gln Lys Met Gln)ₙ-Ala Met Met Gln Ala-
   soit
   -AKKKSK-(QKMQ)ₙ-AMMQA-
   séquence n° 17 : -Ala Met Met-
   soit
   -AMM-
séquence n° 18 : -Ala Lys Lys Ala Met Met-
   soit
   -AKKAMM-
séquence n°19 : -Ala Met Met Gln Ala-
   soit
   -AMMQA-
séquence n° 20 : -Ala Lys Lys Ala Met Met Gln Ala-
   soit
   - AKKAMMOA-
séquence n° 21 : -Pro Gly Asn Ser-
   soit
   -PGNS-

Z peut aussi représenter une construction mixte de formules III à V. dans lesquelles m est un nombre entier de 1 à 10.

A titre d'exemple Z peut ainsi répondre aux séquences suivantes :
séquence n° 22 : -Ala Lys Lys-NH-(CH₂)ₘ-CO-
   soit
   -AKK-NH-(CH₂)ₙ-CO-
séquence n° 23 : -NH-(CH₂)ₘ-CO-Ala Met Met-
   soit
   -NH-(CH₂)ₘ-CO-AMM-
séquence n° 24 : -Ala Lys Lys-NH-(CH₂)ₘ-CO-Ala Met Met-
   soit
   -AKK-NH-(CH₂)ₘ-CO-AMM-
séquence n° 25 : -NH-(CH₂)ₘ-CO-Ala Met Met Gln Ala-
   soit
   -NH-(CH₂)ₘ-CO-AMMQA-
séquence n° 26 : -Ala Lys Lys-NH-(CH₂)ₘ-CO-Ala Met Met Gln Ala-
   soit
   -AKK-NH-(CH₂)ₘ-CO-AMMQA-

Lorsque Ξ et/ou Ψ comportent une séquence peptidique, cette séquence peut former un épitope étendu avec E₁ et/ou E₂ avec lesquels elle est reliée.

De manière avantageuse, la structure chimique de Ξ est telle qu'elle permet la fixation des composés biépitopiques de l'invention à une protéine porteuse naturelle, à une construction peptidique ou à une phase solide.

Les peptides synthétiques biépitopiques de formule Ia et Ib font partie des peptides de la formule I

Ξ-Thr Glu Pro His-Z-Leu Leu Gly Ala Arg-Ψ (Ia)

Ξ-Pro Ala Pro Ile Arg Arg Arg-Z- Thr Glu Pro His-Ψ (Ib)

Dans les formules Ia et Ib :
- Z inclut ou représente une séquence choisie parmi les séquences suivantes :
   séquence n° 6: -Gln Lys Met Gln-
   séquence n° 7: -Gln Gly Pro Asp Asn-
   séquence n° 8 : -Gly Pro Asp Asn-
   séquence n° 20 : -Ala Lys Lys Ala Met Met Gln Ala-
   séquence n° 26 : -Ala Lys Lys-NH-(CH₂)ₘ-CO-Ala Met Met Gln Ala
   séquence n° 12 : -Ala Lys Lys Ala Met Met Lys Ser Lys-(Gln Lys Met Gln)ₙ-Gln Ala-
   séquence n° 16 : -Ala Lys Lys Lys Ser Lys-(Gln Lys Met Gln)ₙ-Ala Met Met Gln Ala-
   et séquence n° 21 : -Pro Gly Asn Ser-,
   dans lesquelles n représente un nombre entier de 1 à 5 et m, un nombre entier de 1à10,
- Ξ représente un radical acétyl, un radical cystéinyle, un radical biotinyle ou biocytinyle éventuellement lié avec le reste d'une séquence peptidique comprenant une séquence choisie parmi les séquences suivantes :
   - Gly Asn Tyr Arg Ala Tyr Ala-
   - Gly Gly Asn Tyr Arg Ala Tyr Ala-
   - Asn Tyr Arg Ala Tyr Ala-, et
   - Arg Pro Ala-
      et
- Ψ représente un radical amino ou une séquence peptidique comprenant une des séquences suivantes :
   - Ala Lys Glu
   - Ala Lys Lys Lys Ser Lys

On décrit tout peptide incluant l'une des séquences 27 à 33 qui sont les suivantes :
séquence n° 27
séquence n° 28
séquence n° 29
séquence n° 30
séquence n° 31
séquence n° 32
séquence n° 33

Les peptides synthétiques biépitopiques de formule Ia et Ib ci-après sont également décrits. Dans les deux tableaux qui suivent, les séquences peptidiques sont données en utilisant le code à une lettre.

***Ξ-TEPH-Z-LLGAR-Ψ*** **(Ia)**

| **COMPOSE** | Ξ- | -Z- | -Ψ |
|---|---|---|---|
| 1 | Ac- | -QKMQ- | -NH₂ |
| 2 | Ac- | -GPDN- | -NH₂ |
| 3 | biotinyl- | -OKMQ- | -NH₂ |
| 4 | biotinyl- | -GPDN- | -NH₂ |
| 5 | Ac-GNYRAYA- | -AKKAMMOA- | -AKE-NH₂ |
| 6 | Ac-GNYRAYA- | *-AKK*-NH-(CH₂)₅-CO-AMM*QA*- | -AKE-NH₂ |
| 7 | Ac-GGNYRAYA- | -*AKK*AMMKSK-(QKMQ)₂-*QA*- | -AKE-NH₂ |
| 8 | Ac-GGNYRAYA- | -*AK*KAMMKSK-(QKMQ)₁-*QA*- | -AKE-NH₂ |
| 9 | Ac-NYRAYA- | -*AKK*AMMKSK-(QKMQ)₁-*QA*- | -AKE-NH₂ |
| 10 | Ac-NYRAYA- | -*AKK*KSK-(QKMQ)₁-AMM*QA*- | -AKE-NH₂ |
| 11 | Ac-GGNYRAYA- | -*AKK*KSK-(QKMQ)₁-AMMQA- | -AKE-NH₂ |
| 12 | Ac-GGNYRAYA- | -*AKK*SK-(QKMQ)₂-AMMQA- | -AKE-NH₂ |
| 13 | Ac-GNYRAYA- | -*AKK*KSK-(QKMQ)₂-AMMQA- | -AKE-NH₂ |
| 14 | CGNYRAYA- | -*AKK*-NH-(CH₂)₅-CO-AMMQA- | -AKE-NH₂ |

***Ξ-PAPIRRR-Z-TEPH-Ψ*** **(Ib)**

| **COMPOSE** | Ξ- | -Z- | -ψ |
|---|---|---|---|
| 15 | Ac-RPA | -PGNS- | -AKKKSK-NH₂ |

Dans les formules Ia et Ib, les séquences d'acides aminés, soulignées correspondant à E₁ et E₂, représentent un épitope minimum de la troponine I. Les séquences d'acides aminés soulignées de Z forment avec E₁ ou E₂, auxquels elles sont reliées, un épitope étendu de la troponine I.

Parmi les composés indiqués ci-dessus les composés 5, 6, 10, 11, 12, 13, 14 et 15 sont plus particulièrement décrits.

Ces peptides ont les séquences suivantes :
peptide 5
composé 6
composé 10
peptide 11
peptide 12
peptide 13
peptide 14
peptide 15

L'invention concerne les composés biépitopiques :
composé 6 et
composé 10

Les composés synthétiques biépitopiques sont obtenus par synthèse en phase solide selon des méthodes classiques R.B. Merrifield, *J. Amer. Chem. Soc. (1963), 85, pp. 2149-2154.* R.C. Sheppard, *in « Peptides 1971 », Nesvadba H* (*ed*.) *North Holland. Amsterdam, pp. 111 ;* E. Atherton and R.L. Sheppard. *in « Solid phase peptide synthesis, a practical approach », IRL PRESS. (1989), Oxford University Press. pp. 25-34*. Comme synthétiseur automatique peut être utilisé le synthétiseur « 9050 Plus Pep Synthesizer » de Millipore ou un synthétiseur équivalent.

Le support solide utilisé pour les synthèses doit être compatible avec la technique et la chimie utilisée. Par exemple, pour une synthèse sur le synthétiseur « 9050 Plus pep. Synthesizer » il est recommandé d'utiliser une résine adaptée à la technique dite « en flot continu » ; les résines PEG PS répondent à ces critères. Ces supports sont constitués d'un bras (« spacer ») à base de polyéthylène glycol (PEG) situé entre le groupement fonctionnel du polystyrène des billes et le point d'accrochage du premier acide aminé. La nature de ce point d'ancrage peut varier selon la fonction C-terminale choisie. Par exemple, pour un peptide sous forme d'amide, on pourra prendre une résine de type PAL PEG PS.

La résine de départ et les acides aminés utilisés comme matière première sont des produits disponibles dans le commerce (PerSeptive-Biosystem).

Les groupements protecteurs de chaînes latérales suivants ont été utilisés :

| **Aminoacides** | **Groupement protecteurs** |
|---|---|
| Arginine | Pentaméthyl-2,2,4,6,7-dihydrobenzofuran-5-sulfonyle (Pbf) |
| Asparagine. Glutamine | Trityle (Trt) |
| Acide glutamique | Ester tert-butyle (otBu) |
| Thréonine. Tyrosine | Ether tert-butyle (tBu) |
| Lysine | Tert-butyloxycarbonyle (Boc) |

La protection temporaire de la fonction amine primaire en α des aminoacides a été effectuée à l'aide du groupement 9-fluorénylméthyloxylcarbonyle (Fmoc). La déprotection est effectuée par une solution de pipéridine à 20% dans le diméthylformamide.

Pour le couplage, on utilise de préférence un excès de diisopropylcarbodiimide (DIPCDI) et d'hydroxy-1-benzotriazole (HOBt).

Après synthèse, la résine est lavée avec des solvants organiques, (diméthylformamide puis dichlorométhane) séchée sous vide puis traitée par une solution à base d'acide trifluoroacétique refroidie à 0°C et contenant des « scavengers » appropriés. On pourra utiliser, par exemple, le réactif K contenant 82% d'acide trifluoroacétique, 5% de phénol, 5% d'eau, 5% de thioanisole et 3% d'éthanedithiol.

Les peptides synthétiques biépitopiques ainsi isolés sont ensuite précipités et rincés à l'éther.

Les composés synthétiques biépitopiques sont ensuite purifiés par chromatographie liquide en phase inverse et leur pureté est déterminée par spectrométrie de masse. Comme phase, on peut utiliser par exemple la phase Bondapak C-18. Les peptides sont élués en réalisant un gradient linéaire entre deux solutions tampons, le premier essentiellement aqueux (par exemple eau-TFA 0.1 %) et le second plutôt organique (par exemple un mélange contenant de l'acétonitrile 60 %, eau 40 % et TFA 0,08 %). Les fractions pures collectées sont rassemblées, concentrées sous vide et lyophylisées.

La détermination du caractère bièpitopique des composés de formule I a été effectuée en utilisant une trousse de dosage de la troponine 1. On peut utiliser par exemple une trousse permettant de mettre en oeuvre un immunodosage de type sandwich et contenant des anticorps monoclonaux qui réagissent avec les séquences peptidiques E₁ et E₂.

Pour déterminer le caractère biépitopique, les composés de formule 1 sont dilués dans le diluant de la trousse, qui peut être par exemple du sérum humain normal ou une solution tampon, et dosés comme un échantillon de patient.

L'utilisation du composé 6 et du composé 10 comme étalon pour le dosage immunologique de la troponine 1 fait également partie de l'invention.

La présente invention concerne également des compositions contenant le composé 6 ou le composé 10. Il s'agit de préférence de solutions aqueuses ou de compositions comprenant le composé 6 ou le composé 10 dans une solution tampon. Comme solution tampon, on peut utiliser, par exemple, une solution tampon phosphate (KH₂PO₄/K₂HPO₄ pH = 6,5 - 7,5) contenant du Kathon et du BSA ou du Kathon, du Régilait et d'EDTA, ou du Kathon, du Plasmion et éventuellement de l'EDTA, ou du Kathon, de la caséine et de l'EDTA.

On peut utiliser également des solutions tampon succinate (pH = 5 - 6) ou Tris HCl (pH = 7,5 - 8,5) contenant du Kathon et du BSA ou du Kathon, du Régilait et de l'EDTA ou du Kathon, du Plasmion et éventuellement de l'EDTA, ou du Kathon, de la Caséine et de l'EDTA.

Des solutions tampons contenant de la glycine, du Kathon, du Régilait et de l'EDTA peuvent être également utilisées.

On préfère les solutions tampon succinate ou phosphate contenant du Kathon, du Régilait et de l'EDTA.

Le Kathon^{®} agent anti-bactérien commercialisé par la Société Rhom et Hass, est constitué,de 5-chloro 2-méthyl 4-isothiazolin-3-one et de 2-méthyl 4-isothiazolin-3-one (1,5%).

Le Régilait^{®} est commercialisé par la société Régilait.

Le Plasmion^{®} est commercialisé par les laboratoires Bellon et est constitué de gélatine fluide modifiée (30 g/l), NaCl (5.382 g/l), MgCl (143 mg/l), KCl (373 mg/l), lactate de sodium (3,360 g/l), dans l'eau.

Les solutions tampons suivantes sont particulièrement préférées
- solution tampon succinate 0,1 M (pH = 6) contenant du Kathon (0.2%), du Régilait (0,05 2%) et de l'EDTA 2mM,
- solution tampon succinate 0,1M (pH = 6) contenant du Kathon (0.2%) de la caséine (0,01 - 0,5%) et de l'EDTA 2mM,
- solution tampon phosphate KH₂PO₄/K₂HPO₄ 0.1 M (pH = 7.5) contenant du Kathon (0.2%) du Régilait (0,05 - 0,5%) et de l'EDTA 2mM,
- solution tampon phosphate KH₂PO₄/K₂PO₄ 0,1 M (pH = 7,5) contenant du Kathon (0.2%), de la caséine (0,01 - 0,1%) et de l'EDTA 2mM.

Des compositions contenant du plasma et un composé 6 ou un composé 10, font aussi partie de la présente invention.

Des procédés d'immunodosages utilisant comme étalons ou témoins le composé 6 ou le composé 10 font également partie de l'invention.

L'invention vise aussi des trousses pour la mise en oeuvre d'immunodosages qui incluent un composé 6 ou un composé 10 ou une composition qui contient un de ces composés.

### EXEMPLE 1 :

### Préparation d'un composé selon l'invention (peptide 10)

Ce peptide a été synthétisé en phase solide. La technique mise au point en 1963 par Merrifield (J. *Am. Chem. Soc. (1963) 85 pp. 2149-2154)* consiste à fixer le premier aminoacide sur un support solide polymérique (résine) par sa fonction acide et à allonger la séquence peptidique à partir de ce premier acide aminé, le peptide en cours de synthèse restant ancré sur la résine.

Pour la synthèse du composé 10. ont été utilisés comme synthétiseur, le synthétiseur « 9050 Plus Pep Synthetiser » et comme résine, la résine PEG PS décrite précédemment.

Les différentes étapes de la synthèse sont résumées dans le tableau I

**Tableau I**

| **RESlDU AMINOACIDE** | **PROTECTION NH₂** | **PROTECTION LATERALE** | **METHODE DE COUPLAGE** | **NBRE d'eq- DUREE (DC)** |
|---|---|---|---|---|
| Glu | Fmoc | otBu | DIPCDI/HOBt | 5 eq - 30 min |
| Lys | Fmoc | Bac | DIPCDI/HOBt | 5 eq - 30 min |
| Ala | Fmoc | | DIPCDI/HOBt | 5 eq - 30 min |
| Arg | Fmoc | Pbf | DIPCDI/HOBt | 5 eq - 30 min |
| Ala | Fmoc | | DIPCDI/HOBt | 5 eq - 30 min |
| Gly | Fmoc | | DIPCDI/HOBt | 5 eq - 30 min |
| Leu | Fmoc | | DIPCDI/HOBt | 5 eq - 30 min |
| Leu | Fmoc | | DIPCDI/HOBt | 5 eq - 30 min |
| Ala | Fmoc | | DIPCDI/HOBt | 5 eq - 30min |
| Gln | Fmoc | Trt | DIPCDI/HOBt | 5 eq - 30 mm |
| Met | Fmoc | | DIPCDI/HOBt | 5 eq - 30 min |
| Met | Fmoc | | DIPCDI/HOBt | 5 eq - 30 min |
| Ala | Fmoc | | DIPCDI/HOBt | 5 eq - 30 min |
| Gln | Fmoc | Trt | DIPCDI/HOBt | 5 eq - 30 min |
| Met | Fmoc | | DIPCDI/HOBt | 5 eq - 30 min |
| Lys | Fmoc | Boc | DIPCDI/HOBt | 5 eq - 30 min |
| Gln | Fmoc | Trt | DIPCDI/HOBt | 5 eq - 30 min |
| Lys | Fmoc | Boc | DIPCDI/HOBt | 5 eq - 30 min |
| Ser | Fmoc | tBu | DIPCDI/HOBt | 5 eq - 30 min |
| Lys | Fmoc | Boc | DIPCDI/HOBt | 5 eq - 30 min |
| Lys | Fmoc | Boc | DIPCDI/HOBt | 5 eq - 30 min |
| Lys | Fmoc | Boc | DIPCDI/HOBt | 5 eq - 30 min |
| Ala | Fmoc | | DIPCDI/HOBt | 5 eq - 30 min |
| His | Fmoc | Trt | DIPCDI/HOBt | 5 eq - 30 min |
| Pro | Fmoc | | DIPCDI/HOBt | 5 eq - 30 min |
| Glu | Fmoc | otBu | DIPCDI/HOBt | 5 eq - 30 min |
| Thr | Fmoc | tBu | DIPCDI/HOBt | 5 eq - 30 min |
| Ala | Fmoc | | DIPCDI/HOBt | 5 eq - 30 min |
| Tyr | Fmoc | tBu | DIPCDI/HOBt | 15 eq - 30 min |
| Ala | Fmoc | | DIPCDI/HOBt | 5 eq - 30 min |
| Arg | Fmoc | Pbf | DIPCIDI/HOBt | 5 eq - 30 min |
| Tyr | Fmoc | tBu | DIPCDI/HOBt | 5 eq - 30 min |
| Asn | Fmoc | Trt | DIPCDI/HOBt | 5 eq - 30 min |
| Acétyl | | | (CH₃CO)₂O/DIEA | 5 eq - 30 min |

Après la fin de la synthèse, la résine a été lavée avec du diméthylformamide, puis du dichlorométhane et séchée sous vide.

Ensuite, la résine a été traitée avec du réactif K (acide trifluoroacétique 82 % ; phénol 5 % ; eau 5 % ; thioanisole 5 %: éthanedithiol 3 %). Le composé 10 ainsi isolé par précipitation, a été ensuite rincé au diéthyle oxyde. On a ainsi obtenu 0,529 g du composé 10.

De manière équivalente, et en utilisant les acides aminés appropriés, ont été synthétisés d'autres composés.

Le poids moléculaire, évalué par spectrométrie de masse de certains composés de formule Ia et Ib sont donnés ci-après.

| **Composé** | **Poids moléculaire (Dalton)** |
|---|---|
| | |
| 1 | 1549,8 |
| 2 | 1545,5 |
| 5 | 3018 |
| 6 | 3132,5 |
| 7 | 4448 |
| 8 | 3934,5 |
| | |
| 9 | 3820,5 |
| 10 | 3820,5 |
| 11 | 3934,5 |
| 12 | 4448 |
| 13 | 4391 |
| 15 | 2721 |

### EXEMPLE 2 :

### Evaluation d'immunoréactivité des peptides

La détermination du caractère biépitopique des composés mentionnés ci-dessus a été réalisée en utilisant une trousse de dosage de la troponine I. Il s'agit de la trousse ERIA Troponine I Pasteur Code 79691.

Son principe est basé sur une méthode immunoenzymatique de type sandwich qui permet un dosage quantitatif de la troponine I cardiaque dans le sérum humain.

La phase solide est constituée de tubes en polystyrène revètus d'un anticorps monoclonal anti-troponine I cardiaque (8E1).

La révélation est réalisée à l'aide d'un deuxième anticorps monoclonal anti-troponine 1 cardiaque (11E12) couplé à la peroxydase.

La mise en oeuvre du test comprend les étapes suivantes :
Les échantillons et les étalons (étalons de troponine 1 d'origine animale) ainsi que l'anticorps monoclonal couplé à la peroxydase sont incubés en présence de l'anticorps monoclonal anti-troponine I cardiaque immobilisé sur la phase solide.

Après une série de lavages, on procède à la révélation enzymatique par addition du chromogène tétraméthylbenzidine.

Après arrêt de la révélation, la lecture de la densité optique est faite à λ = 450 nm. L'absorbance obtenue est directement corrélée à la concentration en troponine l cardiaque présente dans un tube.

Les composés 1, 2, 5, 6, 10, 11 et 12 ont été mis en solution dans l'eau (C = 1 mg/ml ou 2 mg/ml selon le cas). Ces solutions ont été rediluées en utilisant un tampon succinate 0,1 M pH = 6 contenant du Régilait 0,05% et du Kathon 0.2% et de l'EDTA 2mM. Les « solutions finales » ont ensuite été dosées comme « échantillons » selon le protocole décrit ci-dessus.

Par ailleurs, les composés 1, 2, 10 et 11 ont été testés après dilution dans le sérum humain. Dans ce cas, les « solutions finales » ont été obtenues en diluant dans du sérum humain normal, des solutions aqueuses « mères » contenant les composés 1, 2, 10 et 11 à une concentration de 2mg/ml.

Tous les composés sont réactifs dans le dosage de la troponine I.

### EXEMPLE 3 :

### Tests de stabilité

Un composé selon l'invention (composé 10) a été dilué dans l'eau (C = 2mg/ml). A partir de cette solution « mère » sont obtenues quatre solutions « filles » S₁. S₂, S₃ et S₄.

Pour effectuer les différents dilutions, a été utilisée pour les solutions S₁ (C = 0,5ng/ml) et S₂ (C = 1ng/ml), une solution tampon succinate de sodium 0,1 M (pH = 6) contenant du Kathon (0,2%), du Régilait (0,05%) et de l'EDTA 2mM.

Les solutions S₃ (C = 0,5ng/ml) et S₄ (C = 1ng/ml) ont été obtenues en diluant la solution « mère » avec du tampon phosphate (KH₂PO₄/K₂HPO₄ pH = 7,5) contenant du Kathon (0,2%), du Régilait (0,05%) et de l'EDTA 2mM.

Un dosage de solutions S₁, S₂, S₃ et S₄ selon le protocole décrit dans l'exemple 2 a été effectué à J0 (jour de la préparation des solutions). Les valeurs obtenues ont servi de référence pour le suivi des stabilités.

Ensuite, les solutions S₁, S₂, S₃ et S₄ ont été conservées à + 4°C et dosées périodiquement. Lors de chaque série de dosages, ont été utilisées trois « solutions contrôles ». Il s'agit de sérums de contrôle de la Troponine 1 lyophilisés répartis dans la gamme de mesure du dosage. Pour ces sérums, il a été préalablement démontré que la conservation d'un sérum de contrôle lyophilisé est supérieur à 18 mois. Lors de chaque test de stabilité, il a été vérifié que chacun des contrôles testés était obtenu à sa concentration cible. Ceci a donc permis de comparer les concentrations obtenues pour les peptides lors des différents tests. En particulier, il a été possible de comparer la concentration de la solution du composé selon l'invention à J0 (jour auquel a été effectuée la solution) avec la concentration du composé selon l'invention lors des essais de la stabilité au cours du temps. Le fait de toujours retrouver, pour les contrôles, une valeur constante au cours des essais de stabilité permet de valider les tests effectués.

Les résultats obtenus lors des essais de stabilité sont indiqués dans les tableaux II et III.

**Tableau II**

| **Solutions** | **Absorbance (λ = 450 nm)** | | | | |
|---|---|---|---|---|---|
| | J0 | J + 1 mois | J + 2 mois | J + 3 mois | J + 4 mois |
| Blanc (C = 0 ng/ml) | 0,00 | 0,00 | 0,01 | 0,00 | 0,03 |
| S₁ (C = 0,5 ng/ml) | 0,42 | 0,038 | 0,44 | 0,40 | 0,40 |
| S₂ (C = 1,0 ng/ml) | 1,05 | 0,90 | 0,87 | 0,95 | 1,10 |
| S₁ (C = 0,5 ng/ml) | | 0,90 | 1,05 | 0,95 | 0,95 |
| S₂ (C = 1,0 ng/ml) | | 0,86 | 0,83 | 0,90 | 1,05 |

**Tableau III**

| **Solutions** | **Absorbance (λ** = **450 nm)** | | | | |
|---|---|---|---|---|---|
| | J0 | J + 1 mois | J + 2 mois | J + 3 mois | J + 4 mois |
| Blanc (C = 0 ng/ml) | 0,00 | 0,00 | 0,00 | 0,00 | 0,00 |
| S₃ (C = 0,5 ng/ml) | 0,27 | 0,23 | 0,27 | 0,25 | 0,28 |
| S₄ (C = 1,0 ng/ml) | 0,71 | 0,59 | 0,68 | 0,63 | 0,63 |

| | | J + 1 mois/JO | J + 2 mois/JO | J + 3 mois/JO | J + 4 mois/JO |
|---|---|---|---|---|---|
| S₃ (C = 0,5 ng/ml) | | 0,85 | 1,00 | 0,93 | 1,04 |
| S₄ (C = 1,0 ng/ml) | | 0,83 | 0,96 | 0,89 | 0,89 |

Les résultats indiqués dans les tableaux I et Il démontrent l'excellente stabilité des solutions du composé 10 selon l'invention. 6

Des essais effectués avec le composé 6 ont démontré que sa stabilité est comparable à celle du composé 10. Par exemple, ces essais effectués avec le composé 6 ont confirmé que ce composé avait une excellente stabilité pendant au moins 9 mois, lorsque conservé à +4°C en solution dans une solution tampon succinate de sodium 0,1M (pH=6) contenant du Kathon (0,2%), du Régilait (0,2%) et de l'EDTA 2mM. Le composé 6 a été dissous dans le tampon indiqué ci-dessus pour obtenir des concentrations de 0,25, 4,8 et 18 ng/ml. Les résultats de cet essai sont donnés dans le tableau IV.

Cette étude de stabilité a été validée à l'aide du composé 6 conservé à -20°C et utilisé comme référence.

Il a été préalablement vérifié que le composé 6 est stable lorsque conservé à -20°C pendant au moins 18 mois.

**Tableau IV**

| **Solutions** | **Concentration en ng/ml** | | | | | | | |
|---|---|---|---|---|---|---|---|---|
| | J0 | J+1 mois | J+2mois | J+3mois | J+4mois | J+5mois | J+6mois | J+9mois |
| Solution 1 | 0.26 | 0.29 | 0.24 | 0.24 | 0.23 | 0.25 | 0.23 | 0.22 |
| Solution 2 | 4.93 | 5.25 | 4.58 | 4.77 | 4.57 | 4.42 | - | 3.87 |
| Solution 3 | 18.21 | 19.61 | 16.79 | 17.48 | 16.35 | 17.21 | 16.13 | 15.04 |

## Revendications

1. Composé biépitopique choisi parmi les composés suivants :
composé 6 et
composé 10

2. Utilisation d'un composé biépitopique selon la revendication 1 comme étalon dans un immunodosage de la troponine 1.

3. Composition contenant un composé selon la revendication 1 en solution dans l'eau, dans le plasma ou dans une solution tampon.

4. Composition selon la revendication 3 **caractérisée en ce que** la solution tampon est choisie parmi les solutions tampon phosphate, succinate ou Tris HCl.

5. Composition selon les revendications 3 et 4 **caractérisée en ce que** la solution tampon est une des solutions suivantes :
- solution tampon succinate (pH = 5 - 6) contenant du Kathon®, du Régilait® et de l'EDTA,
- solution tampon succinate (pH = 5 - 6) contenant du Kathon®, de la caséine et de l'EDTA,
- solution tampon phosphate (KH₂PO₄/K₂HPO₄ pH = 6,5 - 7,5) contenant du Kathon®, du Régilait® et de l'EDTA,
- solution tampon phosphate (KH₂PO₄/K₂HPO₄ 0,1 M pH = 6,5 - 7,5) contenant du Kathon®, de la caséine et de l'EDTA.

6. Procédé d'immunodosage utilisant comme étalon ou témoin un composé biépitopique selon la revendication 1.

7. Procédé selon la revendication 6 **caractérisé en ce que** l'immunodosage est de type sandwich.

8. Trousse pour la mise en oeuvre d'immunodosage incluant un peptide biépitopique, selon la revendication 1, ou une composition contenant un composé biépitopique selon l'une quelconque des revendications 3 à 5.

## Claims

1. Biepitope compound chosen from the following compounds:
compound 6 and
compound 10

2. The use of a biepitope compound according to claim 1 as a standard in immunoassay of troponin 1.

3. Composition containing a compound according to claim 1 in solution in water, in plasma or in a buffer solution.

4. Composition according to claim 3 **characterised by** the fact that the buffer solution is chosen from phosphate, succinate or Tris HCl buffer solutions.

5. Composition according to claims 3 and 4 **characterised by** the fact that the buffer solution is one of the following solutions:
- succinate buffer solution (pH = 5 - 6) containing Kathon®, Regilait® and EDTA,
- succinate buffer solution (pH = 5 - 6) containing Kathon®, casein and EDTA,
- phosphate buffer solution (KH₂PO₄/K₂HPO₄ pH = 6.5 - 7.5) containing Kathon®, Regilait® and EDTA,
- phosphate buffer solution (KH₂PO₄/K₂HPO₄ 0.1 M pH = 6.5 - 7.5) containing Kathon®, casein and EDTA.

6. Immunoassay procedure using as a standard or control a biepitope compound according to claim 1.

7. Procedure according to claim 6 **characterised by** the fact that the immunoassay is of the sandwich type.

8. Kit for use for immunoassay which includes a biepitope peptide according to claim 1, or a composition containing a biepitope compound according to any of claims 3 to 5 whatsoever.

## Patentansprüche

1. Biepitop-Verbindung, die aus folgenden Verbindungen ausgewählt ist:
Verbindung 6 und
Verbindung 10

2. Verwendung einer Biepitop-Verbindung nach Anspruch 1 als Standard in einem Immunoassay für Troponin I.

3. Zusammensetzung, die eine Verbindung nach Anspruch 1 in Wasser, Plasma oder einer Pufferlösung gelöst enthält.

4. Zusammensetzung nach Anspruch 3, **dadurch gekennzeichnet, dass** die Pufferlösung aus einer Phosphat-, Succinat- oder Tris-HCl-Pufferlösung ausgewählt ist.

5. Zusammensetzung nach den Ansprüchen 3 und 4, **dadurch gekennzeichnet, dass** die Pufferlösung eine der folgenden Lösungen ist:
- Succinatpufferlösung (pH = 5 bis 6), die Kathon®, Regilait® und EDTA enthält,
- Succinatpufferlösung (pH = 5 bis 6), die Kathon®, Casein und EDTA enthält,
- Phosphatpufferlösung (KH₂PO₄/K₂HPO₄ pH = 6,5 bis 7,5), die Kathon®, Regilait® und EDTA enthält, und
- Phosphatpufferlösung (KH₂PO₄/K₂HPO₄ 0,1 M, pH = 6, 5 bis 7,5), die Kathon®, Casein und EDTA enthält.

6. Immunoassayverfahren, in welchem als Standard oder Kalibriersubstanz eine Biepitop-Verbindung nach Anspruch 1 verwendet wird.

7. Verfahren nach Anspruch 6, **dadurch gekennzeichnet, dass** der Immunoassay ein Sandwich-Immunoassay ist.

8. Kit für die Durchführung des Immunoassays, welcher ein Biepitop-Peptid nach Anspruch 1 oder eine Zusammensetzung, die eine Biepitop-Verbindung nach einem der Ansprüche 3 bis 5 enthält, umfasst.
